# FASCICULE DE BREVET EUROPEEN

(11) **EP 1 490 134 B1**
(45) Date de publication et mention de la délivrance du brevet: **14.05.2008**
(21) Numéro de dépôt: 03712477.3
(22) Date de dépôt: 02.04.2003
(51) Int. Cl.: A61M 5/34, A61C 3/02

(54) **PORTE-OUTIL POUR OUTIL DEFORMABLE EN FLEXION**
WERKZEUGHALTER FÜR EIN DURCH BIEGUNG DEFORMIERBARES WERKZEUG
TOOL HOLDER FOR FLEXIBLY-DEFORMABLE TOOL

(30) Priorité: 02.04.2002 FR 0204093
(43) Date de publication de la demande: 29.12.2004
(73) Titulaire: Primequal S.A., 1268 Begnins (CH)
(72) Inventeur: Delval, Alain, 1348 Le Brassus (CH); Rosset, Yannick, 1298 Céligny (CH); Volckmann, Jean-Claude, 74380 Bonne (FR)
(74) Mandataire: Bugnion Genève
(86) Numéro de dépôt international: PCT/IB2003/001192
(87) Numéro de publication internationale: WO 2003/082387

(56) Documents cités:
- FR-A- 2 535 206
- FR-A- 2 785 813
- GB-A- 682 372
- US-A- 2 016 631
- US-A- 3 534 476
- US-B1- 6 200 296

## Description

La présente invention concerne un porte-outil destiné à recevoir un outil déformable en flexion. Elle concerne plus précisément un porte-outil défini selon le préambule de la revendication 1. L'invention concerne aussi un dispositif comprenant un tel porte-outil et un outil déformable en flexion.

On connaît de la demande de brevet FR 2 535 206 une seringue dentaire pour injection intra-ligamentaire. Cette seringue permet l'injection de produit par une aiguille très fine et souple dans les ligaments situés entre l'os de la mâchoire et la dent. Elle est principalement constituée d'un corps allongé sur lequel est articulé un levier commandant l'injection, d'un porte-conteneur dans lequel est logé un conteneur rempli de liquide à injecter et d'un embout comportant l'aiguille d'injection. Afin de résoudre des problèmes d'accès difficile aux zones où doivent être faites les injections, le corps de la seringue présente une tête d'injection qui fait un angle avec l'axe du corps de la seringue. L'aiguille, amovible, est mise en place sur le corps avant de pratiquer les injections puis retirée après. Lors de sa mise en place, l'aiguille est introduite dans le corps de seringue parallèlement à l'axe de la tête d'injection en subissant une flexion pour que son extrémité se place parallèlement à l'axe du porte-conteneur. Le guidage de l'aiguille lors de son introduction et les efforts nécessitant sa flexion sont assurés par un trou conique. L'angle entre l'axe de la tête d'injection et l'axe du corps de seringue étant important, l'aiguille introduite dans la tête d'injection, vient en contact avec une génératrice du cône en formant avec celle-ci un angle trop important et se tord. Pour remédier à ce problème, une demande de certificat d'addition 2 556 595 à un brevet français, prévoit un canal débouchant à l'extrémité de la tête d'injection et dans le porte-conteneur. Ce canal permet le guidage de l'aiguille de son introduction dans la tête d'injection jusqu'à sa sortie dans le porte-conteneur. Cependant, cette solution présente des inconvénients. D'abord, le diamètre de l'aiguille et celui du canal étant sensiblement les mêmes, l'introduction de l'aiguille dans le canal est malaisée voire dangereuse. Des cas de blessure de l'utilisateur par l'introduction manquée de l'aiguille ont été relevés. Ensuite, les seringues présentent au niveau de la fixation de l'aiguille des zones difficiles à nettoyer, à désinfecter donc difficile à stériliser. C'est en particulier le cas pour le canal courbé, le pourtour du cône et l'arrière de la tête d'injection. Enfin, la réalisation d'une seringue comportant un canal courbé est compliquée et, par conséquent, d'un coût élevé.

Pour remédier à ces problèmes, on connaît de la demande FR 2 785 813 une seringue d'injection qui présente à l'extrémité de son corps un canal destiné à recevoir une aiguille et ayant une partie conique dont l'axe est parallèle à l'axe du corps et qui s'évase vers l'extérieur du corps. L'aiguille est liée à une bague taraudée venant se visser sur une tête d'injection filetée présentant un angle avec l'axe du corps. Cette seringue présente des inconvénients. D'abord, la partie conique interfère avec la tête filetée si bien que le filetage est inexistant sur une portion de la tête. Ceci nuit à la qualité de la liaison entre le corps et l'aiguille. Ensuite, lorsqu'on introduit l'aiguille selon une direction parallèle à l'une des génératrices du cône, le biseau à l'extrémité de l'aiguille risque de venir buter contre la génératrice opposée du cône. Ceci engendre des risques de blessures de l'utilisateur qui tente de positionner l'aiguille dans le canal. Enfin, l'aiguille, une fois courbée dans sa direction de travail, est en appui sur une des génératrices de la partie conique et risque de ce fait d'être endommagée. En effet, compte tenu de la qualité de la surface de la partie conique, l'aiguille porte non sur la totalité d'une génératrice, pendant son mouvement de fixation au corps, mais sur quelques points. La pression exercée par ces points sur l'aiguille risque, compte tenu de ses dimensions, d'endommager non seulement sa partie externe mais aussi son canal d'injection de produit. Un tel endommagement a nécessairement des conséquences sur l'effort qu'il faudra exercer sur la seringue pour injecter le produit qu'elle contient.

La solution de ce problème est achevée par les caractéristiques figurant dans la partie caractérisante de la revendication 1.

L'invention telle que revendiquée a pour but de réaliser un porte-outil et un dispositif comprenant un porte-outil et un outil déformable en flexion palliant à ces inconvénients et améliorant les dispositifs connus de l'art antérieur. En particulier, l'invention se propose de réaliser un dispositif sûr, pouvant être nettoyé et désinfecté facilement, présentant une géométrie simple permettant de minimiser les coûts de fabrication et permettant une liaison de qualité entre l'aiguille et le corps du porte-outil, un bon guidage de l'aiguille lors de son introduction dans le corps et de minimiser les risques d'endommagement de l'aiguille lors de sa liaison au corps.

Le porte-outil selon l'invention est caractérisé par la partie caractérisante de la revendication 1.

Les revendications 2 à 7 définissent des variantes du porte-outil.

Le dispositif comprenant un tel porte-outil et un outil déformable en flexion est défini par la revendication indépendante 8.

Les revendications 9 à 11 définissent des variantes du dispositif.

Le dessin annexé représente, à titre d'exemples, deux modes d'exécution de l'invention.
La figure 1 représente une vue d'ensemble d'un dispositif selon l'invention, comprenant un porte-outil et un outil déformable en flexion.

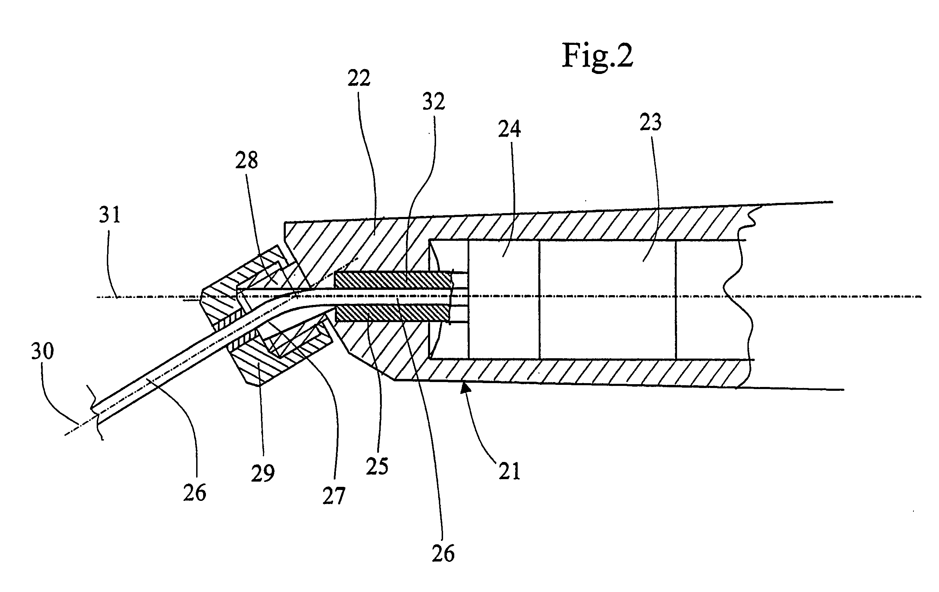

La figure 2 représente une vue schématique en coupe d'un porte-outil selon l'invention, recevant un outil de coupe élastiquement déformable en flexion.
La figure 3 est une vue en coupe du dispositif selon l'invention, l'outil déformable en flexion étant représenté dans deux positions correspondant à deux phases du procédé d'assemblage au porte-outil.

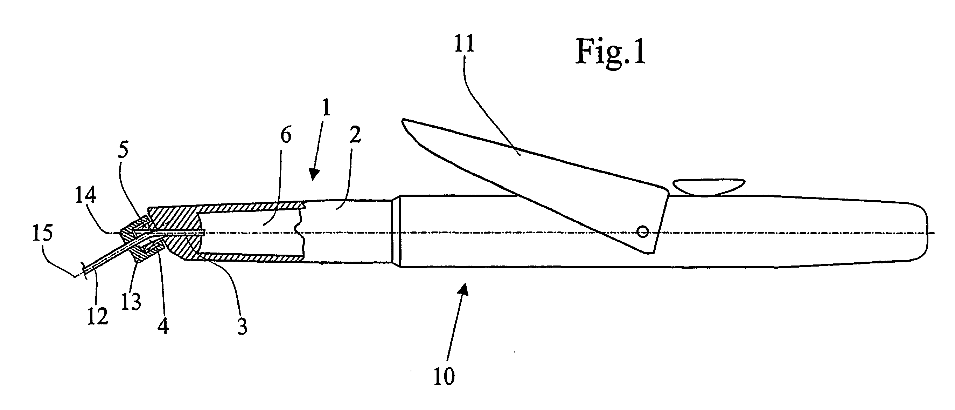

Le dispositif 10 représenté à la figure 1 est une seringue pour injecter des liquides ou des pâtes. Il comprend un porte-outil 1 et un levier 11 agissant sur un mécanisme non représenté commandant le déplacement d'un cylindre de poussée provoquant l'éjection par une aiguille 12 du liquide ou de la pâte. Il est à noter que l'aiguille 12 peut être soit élastiquement déformable en flexion, soit déformable en flexion au-delà du domaine élastique du matériau la constituant. Le porte-outil 1 comprend un corps 2 dans lequel est pratiqué un réservoir 6 pouvant recevoir directement le liquide ou la pâte à injecter ou un conteneur rempli de liquide ou de pâte à injecter. Au fond du réservoir 6, est réalisé un canal comprenant une partie cylindrique 3 parallèle à l'axe du corps 2. Ce canal 3 se termine par une partie 4 en forme de cône divergent débouchant à l'extérieur du corps 2 au niveau du support 5 de l'aiguille d'injection 12. Le support 5 est par exemple fileté et permet de lier l'aiguille 12 au corps 2. La partie conique 4 a la forme d'un cône de révolution dont une des génératrices est sensiblement parallèle à l'axe de la partie cylindrique 3 du canal.

L'aiguille 12 est liée à une bague 13 présentant par exemple un taraudage lui permettant d'être assemblée au support 5 du corps 2.

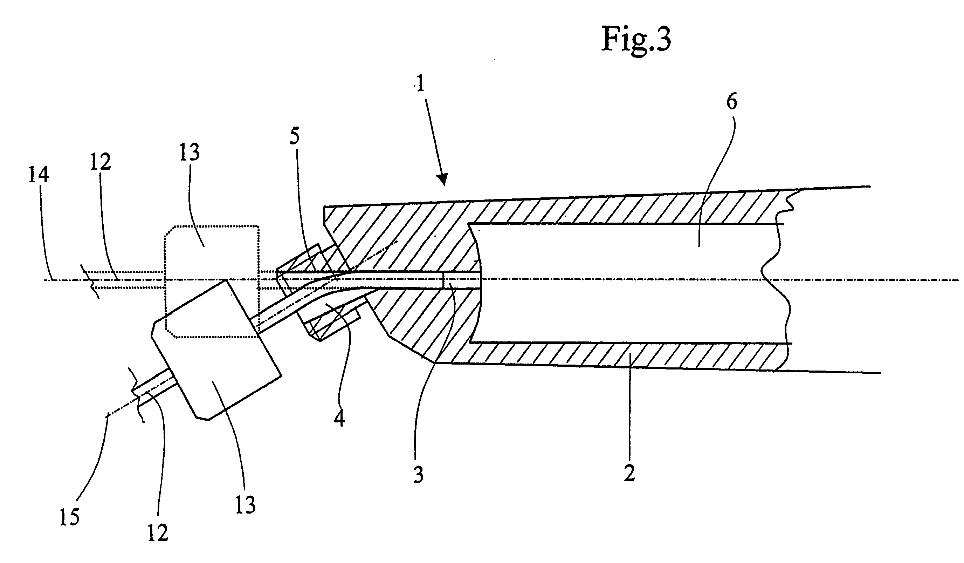

Pour assembler l'aiguille 12 et le corps 2, on introduit l'aiguille 12 dans la partie conique 4. On peut en particulier, afin d'assurer une bonne pénétration de l'aiguille dans la partie cylindrique du canal, appuyer l'aiguille contre le cône au niveau de la génératrice parallèle à l'axe du corps. L'aiguille se trouve ainsi sensiblement parallèle à la partie cylindrique du canal. On introduit ensuite l'aiguille dans la partie cylindrique 3 par un mouvement de translation de celle-ci dans l'axe 14 du corps 2. L'aiguille 12 se trouve alors dans la position représentée en traits pointillés à la figure 3. Cette méthode d'introduction de l'aiguille permet de préserver son extrémité pénétrant dans le corps. On effectue ensuite une flexion de l'aiguille 12 de manière à amener son extrémité libre dans la direction déterminée par l'axe 15 du support 5. L'aiguille 12 se trouve alors dans la position représentée en traits continus à la figure 3. Ensuite, on visse la bague taraudée 13 solidaire de l'aiguille 12 sur le support 5. Lors de cette action, l'aiguille 12 pénètre encore un peu plus dans le canal 3. L'assemblage de l'aiguille au corps se fait donc en trois phases :
- une phase d'introduction de l'aiguille dans le canal,
- une phase de flexion de l'aiguille,
- une phase de fixation de l'aiguille au corps.

Pour enlever l'aiguille 12 du corps 2, on dévisse du support 5, la bague taraudée 13 liée à l'aiguille 12. L'aiguille 12 n'étant plus maintenue en position sur le support 5, on peut la retirer du canal par un mouvement de translation de celle-ci dans l'axe 14 du corps 2.

Grâce à ce procédé d'assemblage, les actions d'introduction de l'aiguille 12 dans le corps 2 et de flexion de l'aiguille 12 s'effectuent l'une après l'autre. Elles sont indépendantes et sont par conséquent plus simples à effectuer.

Il est à noter que le cône 4 peut être remplacé par toute forme permettant d'assurer le guidage de l'aiguille vers la partie cylindrique 3 du canal grâce à une surface dont les génératrices sont sensiblement parallèles à l'axe de cette partie cylindrique et permettant d'assurer que l'aiguille ne vient pas en contact contre cette forme lors de ses phases de flexion et de fixation et lorsqu'elle est en position de travail.

Dans un deuxième mode de réalisation, le corps 22 du porte-outil 21 est appliqué à un dispositif de perçage, de fraisage, de martelage ou de vibration destiné par exemple, au domaine de la chirurgie dentaire. Le corps 22 du porte-outil 21 comprend un moteur pneumatique 23 ou un moteur électrique attaquant un réducteur 24 ou un multiplicateur de vitesse. Il peut aussi comprendre tout type de transducteur permettant de mettre en mouvement, de manière directe ou indirecte, l'outil ou l'ensemble constitué du corps et de l'outil. Le réducteur 24 ou le multiplicateur de vitesse entraîne une broche 25 ayant un axe parallèle au corps 22. La broche 25 est percée par un canal 32 recevant un outil 26 élastiquement déformable en flexion, par exemple, un foret ou une fraise creuse ou non. La broche 25 est en liaison pivot dans le corps 22. L'outil 26 et la broche 25 présentant entre eux un ajustement serré, l'outil 26 est entraîné en rotation par frottement.

La broche se termine par un cône divergent 27 débouchant du corps 22 au niveau du support 28 de l'outil 26. Ce support 28 est fileté et permet de lier l'outil 26 au corps 22. Le cône 27 est un cône de révolution dont une des génératrices est sensiblement parallèle à l'axe de la broche 25.

L'outil 26 est lié par une liaison pivot à une bague 29 assurant son guidage. Cette bague présente un taraudage lui permettant d'être assemblée au support 28 du corps 22.

Pour assembler l'outil 26 et le corps 22, on introduit l'outil 26 dans le cône 27 puis dans la broche 25 par un mouvement de translation de celui-ci selon l'axe 31 du corps 22. Puis, on effectue une flexion de l'outil 26 de manière à amener son extrémité libre dans la direction de travail de l'outil déterminée par l'axe 30 du support. Enfin, on visse la bague taraudée 29 liée à l'outil 26 sur le support 28. Lors de cette action, l'outil 26 pénètre encore un peu plus dans le canal 32 de la broche 25.

Pour enlever l'outil 26 du corps, on dévisse la bague taraudée 29 du support 28. L'outil 26 n'étant plus maintenu en position sur le support, il revient dans sa position initiale rectiligne grâce à ses caractéristiques élastiques. Enfin, on retire l'outil 26 de la broche 25 par un mouvement de translation de celui-ci selon l'axe 31 du corps 22.

Une variante de ce deuxième mode de réalisation diffère du second mode de réalisation en ce que le dispositif comprend une aiguille d'injection à la place de l'outil coupant et en ce qu'il comprend à la place du moteur 23 et du réducteur 24, un dispositif permettant l'orientation de l'aiguille en rotation autour de son axe, par exemple alternativement, permettant ainsi de positionner le biseau de l'aiguille par rapport à la surface à pénétrer pour réaliser l'injection. Un tel dispositif d'orientation d'aiguille est par exemple décrit dans la demande de brevet FR 2 473 318.

Dans les deux modes de réalisation précédemment décrits, la mise en position de l'outil dans le corps du porte-outil se fait par vissage. Cependant, cette fonction peut être assurée par tout autre moyen connu tel un assemblage par baïonnette ou un assemblage par emboîtement. Selon le moyen de fixation retenu, le mouvement de l'aiguille par rapport au corps est différent pendant sa phase de fixation au corps.

## Revendications

1. Porte-outil (1 ; 21) destiné à recevoir un outil déformable en flexion (12 ; 26), comprenant un corps allongé (2; 22), présentant un canal destiné à recevoir l'extrémité de l'outil (12; 26), le canal présentant une partie cylindrique (3 ; 32) parallèle au corps (2 ; 22) et une partie (4 ; 27) débouchant à l'extérieur du corps (2 ; 22), s'évasant vers l'extérieur du corps (2 ; 22) et assurant le guidage de l'outil (12 ; 26) vers la partie cylindrique (3 ; 32) lors de sa mise en place dans le porte-outil (1 ; 21) et des moyens de maintien en position (5, 13 ; 28, 29) de l'outil (12 ; 26) agencés de telle sorte que l'axe de l'outil (12 ; 26) en phase de travail n'est pas parallèle à l'axe du corps (2 ; 22), la partie (4 ; 27) débouchant à l'extérieur du corps (2 ; 22) permettant l'introduction de l'outil (12 ; 26) dans le corps (2 ; 22) par un déplacement de l'outil (12 ; 26) selon l'axe de la partie cylindrique (3 ; 32) du canal, **caractérisé en ce que** la partie (4 ; 27) débouchant à l'extérieur du corps comprend une surface dont une des génératrices est sensiblement parallèle à l'axe de la partie cylindrique du canal et qui s'étend de la partie cylindrique (3 ; 32) à l'extérieur du corps.

2. Porte-outil selon la revendication 1, **caractérisé en ce que** la partie (4 ; 27) débouchant à l'extérieur du corps présente des formes permettant d'éviter son contact avec l'outil pendant ses phases de flexion et de fixation et lorsque celui-ci est en position de travail.

3. Porte-outil selon l'une quelconque des revendications précédentes, **caractérisé en ce que** les moyens de maintien en position (5,13 ; 28, 29) de l'outil (12 ; 26) comprennent sur le corps (2 ; 22), une extrémité filetée (5; 28) sur laquelle se visse une bague taraudée (13 ; 29) liée à l'outil (12 ; 26).

4. Porte-outil selon l'une quelconque des revendications 1 à 2, **caractérisé en ce que** les moyens de maintien en position de l'outil (12 ; 26) comprennent sur le corps, une extrémité coopérant avec une bague liée à l'outil (12 ; 26) pour réaliser un système de liaison à baïonnette.

5. Porte-outil selon l'une quelconque des revendications 1 à 2, **caractérisé en ce que** les moyens de maintien en position de l'outil (12 ; 26) comprennent sur le corps, des moyens de clippage coopérant avec des moyens de clippage complémentaires sur une bague liée à l'outil (12 ; 26).

6. Porte-outil selon l'une quelconque des revendications 1 à 2, **caractérisé en ce que** les moyens de maintien en position de l'outil (12 ; 26) comprennent sur le corps, des moyens d'emboîtement coopérant avec des moyens d'emboîtement complémentaires sur une bague liée à l'outil (12 ; 26).

7. Porte-outil selon l'une quelconque des revendications 1 à 6, **caractérisé en ce qu'**il présente au niveau de la partie cylindrique du canal (3 ; 32) des moyens de guidage en rotation (25) de l'outil (26).

8. Dispositif comprenant un porte-outil (1 ; 21) selon l'une des revendications 1 à 7 et un outil déformable en flexion (12 ; 26) lié à une bague (13 ; 29).

9. Dispositif selon la revendication 8, **caractérisé en ce que** l'outil(12 ; 26) est lié à la bague (29) par une liaison pivot.

10. Dispositif selon l'une des revendications 8 ou 9, **caractérisé en ce que** l'outil (12) est une aiguille d'injection.

11. Dispositif selon la revendication 9, **caractérisé en ce que** le porte-outil (21) présente des moyens d'entraînement en rotation (23, 24, 25) de l'outil (26).

## Claims

1. A tool holder (1; 21) intended to receive a flexibly deformable tool (12; 26), comprising an elongate body (2; 22) with a channel intended to receive the end of the tool (12; 26), the channel having a cylindrical part (3; 32) parallel to the body (2; 22) and a part (4; 27) opening to the outside of the body (2; 22), widening toward the outside of the body (2; 22) and guiding the tool (12; 26) toward the cylindrical part (3; 32) when it is being fitted in the tool holder (1; 21), and means (5, 13; 28, 29) which keep the tool (12; 26) in position and are arranged in such a way that the axis of the tool (12; 26) in the operating phase is not parallel to the axis of the body (2; 22), the part (4; 27) which opens to the outside of the body (2; 22) permitting introduction of the tool (12; 26) into the body (2; 22) by a displacement of the tool (12; 26) along the axis of the cylindrical part (3; 32) of the channel, **characterized in that** the part (4; 27) opening to the outside of the body comprises a surface whose a generatice is substantially parallel to the axis of the cylindrical part of the channel and which extends from the cylindrical part (3; 32) to outside of the body.

2. The tool holder as claimed in claim 1, **characterized in that** the part (4; 27) opening to the outside of the body has configurations allowing it to avoid contact with the tool during its stages of flexion and fixation and when said tool is in the operating position.

3. The tool holder as claimed in either one of the preceding claims, **characterized in that** the means (5, 13; 28, 29) for holding the tool (12; 26) in position comprise, on the body (2; 22), a threaded end (5; 28) onto which an internally threaded ring (13; 29) connected to the tool (12; 26) is screwed.

4. The tool holder as claimed in either one of claims 1 and 2, **characterized in that** the means for holding the tool (12; 26) in position comprise, on the body, an end which cooperates with a ring connected to the tool (12; 26) in order to form a bayonet-type connection system.

5. The tool holder as claimed in either one of claims 1 and 2, **characterized in that** the means for holding the tool (12; 26) in position comprise, on the body, clip means which cooperate with complementary clip means on a ring connected to the tool (12; 26).

6. The tool holder as claimed in either one of claims 1 and 2, **characterized in that** the means for holding the tool (12; 26) in position comprise, on the body, shape-fit means which cooperate with complementary shape-fit means on a ring connected to the tool (12; 26).

7. The tool holder as claimed in any one of claims 1 through 6, **characterized in that**, in the area of the cylindrical part of the channel (3; 32), it has means (25) for guiding the tool (26) in rotation.

8. A device comprising the tool holder (1; 21) as claimed in one of claims 1 through 7 and a flexibly deformable tool (12; 26) connected to a ring (13; 29).

9. The device as claimed in claim 8, **characterized in that** the tool (12; 26) is connected to the ring (29) by a pivot connection.

10. The device as claimed in either of claims 8 and 9, **characterized in that** the tool (12) is an injection needle.

11. The device as claimed in claim 9, **characterized in that** the tool holder (21) has means (23, 24, 25) for driving the tool (26) in rotation.

## Patentansprüche

1. Werkzeughalter (1; 21) zur Aufnahme eines durch Biegung verformbares Werkzeug (12; 26), mit einem länglichen Körper (2; 22), der einen Kanal zur Aufnahme des Endbereichs des Werkzeugs (12; 26) aufweist, welcher einen zum Körper parallelen zylindrischen Bereich (3; 32) (2; 22) und ein Teil (4; 27) enthält, das an der Aussenseite des Körpers (2; 22) ausläuft, sich gegen die Aussenseite des Körpers (2; 22) erweitert und die Führung des Werkzeugs (12; 26) beim Einsetzen des Werkzeugs in den zylindrischen Bereich (3; 32 des Werkzeughalters (1; 21)) sichert, und mit Mitteln zum Festhalten (5, 13; 28, 29) des Werkzeugs (12; 26) an Ort und Stelle, die so eingerichtet sind, dass die Achse des Werkzeugs (12; 26) in Arbeitsstellung zur Achse des Körpers (2; 22) nicht parallel ist, wobei das an der Aussenseite des Körpers (2; 22) auslaufende Teil (4; 27) das Einsetzen des Werkzeugs (12; 26) in den Körper (2; 22) durch Verschieben des Werkzeugs (12; 26) längs der Achse des zylindrischen Bereiches (3; 32) des Kanals ermöglicht, **dadurch gekennzeichnet, dass** das an der Aussenseite des Körpers auslaufende Teil (4; 27) eine Fläche aufweist, bei der eine der Erzeugenden im wesentlichen parallel zur Achse des zylindrischen Bereiches des Kanals verläuft und sich vom zylindrischen Bereich (3; 32) nach der Aussenseite des Körpers erstreckt.

2. Werkzeughalter nach Anspruch 1, **dadurch gekennzeichnet, dass** das an der Aussenseite des Körpers auslaufende Teil (4; 27) eine derartige Form aufweist, dass seine Berührung mit dem Werkzeug während dessen Biegephasen und dessen Befestigung sowie in der Arbeitsstellung des Werkzeugs vermieden wird.

3. Werkzeughalter nach einem der vorstehenden Ansprüche, **dadurch gekennzeichnet, dass** die Mittel zum Festhalten (5, 13; 28, 29) des Werkzeugs (12; 26) einen mit Gewinde versehenen Endbereich (5; 28) des Körpers (2; 22) aufweisen, auf den ein Gewindering (13; 29) aufgeschraubt ist, der mit dem Werkzeug (12; 26) verbunden ist.

4. Werkzeughalter nach einem der Ansprüche 1 und 2, **dadurch gekennzeichnet, dass** die Mittel zum Festhalten des Werkzeugs (12; 26) einen Endbereich des Körpers bilden, der mit einem Ring zusammenwirkt, welcher mit dem Werkzeug (12; 26) verbunden ist, um ein Befestigungssystem nach Art eines Bajonettverschlusses zu bilden.

5. Werkzeughalter nach einem der Ansprüche 1 und 2, **dadurch gekennzeichnet, dass** die Mittel zum Festhalten des Werkzeugs (12; 26) aus Mitteln zum Festklemmen auf dem Körper bestehen, die mit komplementären Mitteln zum Festklemmen auf einem Ring zusammenwirken, der mit dem Werkzeug (12; 26) verbunden ist.

6. Werkzeughalter nach einem der Ansprüche 1 und 2, **dadurch gekennzeichnet, dass** die Mittel zum Festhalten des Werkzeugs (12; 26) Mittel zum Ineinanderstecken auf dem Körper darstellen, die mit komplementären Mitteln zum Ineinanderstecken auf einem Ring zusammenwirken, der mit dem Werkzeug (12; 26) verbunden ist.

7. Werkzeughalter nach einem der Ansprüche 1 bis 6, **dadurch gekennzeichnet, dass** er im Gebiet des zylindrischen Bereiches des Kanals (3; 32) Mittel (25) zur Führung des Werkzeugs (26) durch Drehung aufweist.

8. Vorrichtung, bestehend aus einem Werkzeughalter (1; 21) nach einem der Ansprüche 1 bis 7 und einem durch Biegung verformbaren Werkzeug (12; 26), das mit einem Ring (13; 29) verbunden ist.

9. Vorrichtung nach Anspruch 8, **dadurch gekennzeichnet, dass** das Werkzeug (12; 26) mit dem Ring (29) über eine Drehzapfenanordnung verbunden ist.

10. Vorrichtung nach Anspruch 8 oder 9, **dadurch gekennzeichnet, dass** das Werkzeug (12) eine Injektionsnadel ist.

11. Vorrichtung nach Anspruch 9, **dadurch gekennzeichnet, dass** der Werkzeughalter (21) Mittel (23, 24, 25) aufweist, um das Werkzeug (26) in Drehung zu versetzen.
